# EUROPEAN PATENT APPLICATION

(11) **EP 4 821 545 A1**
(43) Date of publication of application: **14.10.2026**
(21) Application number: 25382365.2
(22) Date of filing: 08.04.2025
(51) Int. Cl.: A61N 5/10

(54) **METHOD AND SYSTEM FOR MEASURING AND/OR VERIFYING THE RADIATION DOSE RECEIVED BY A PATIENT AFTER PARTICLE THERAPY TREATMENT**

(71) Applicant: Administración General de la Comunidad Autónoma de Euskadi, 01010 Vitoria-Gasteiz (Álava) (ES); Universitat Politècnica de València, 46022 Valencia (ES); Consejo Superior de Investigaciones Cientificas, 28006 Madrid (ES)
(72) Inventor: BALCERZYK, Marcin, 01009 Vitoria-Gasteiz (ES); GONZÁLEZ MARTÍNEZ, Antonio Javier, 46022 Valencia (ES); FREIRE LÓPEZ-FANDO, Marta, 46022 Valencia (ES)
(74) Representative: Hoffmann Eitle

(57) **Abstract**

The present invention refers to a method and/or to a system configured to measure and/or verify the radiation dose received by a patient after particle therapy treatment.

## Description

### FIELD OF THE INVENTION

The present invention refers to the medical filed. Particularly, the present invention refers to a method and/or system configured to measure and/or verify the radiation dose received by a patient after particle therapy treatment, preferably after proton therapy and/or therapy treatment with helium ions (He-4).

### STATE OF THE ART

Particle therapy treatment, also known as charged particle therapy, refers to a type of radiation therapy that uses charged particles, such as protons or heavier ions (e.g., helium ions or carbon ions), to treat cancer. Unlike conventional photon therapy (X-rays), particle therapy takes advantage of the unique properties of these particles to deliver highly targeted doses of radiation to tumors while minimizing damage to surrounding healthy tissues.

Particle therapy involves the use of charged particles to irradiate and treat cancer cells. The most common particles used are protons and heavy ions such as helium ions (He-4). These particles have mass and charge, unlike photons, which are electromagnetic radiation. Their charged nature allows them to interact with the target tissue in ways that photons cannot, offering several advantages in terms of precision and biological effectiveness.

Proton therapy uses protons (positively charged particles) to deliver radiation, and it is particularly known for its Bragg peak, a phenomenon where protons deposit the majority of their energy at a specific depth, which allows for minimal damage to healthy tissues outside the tumor.

Helium ion therapy (He-4 therapy) uses helium ions, which are doubly charged, offering a higher linear energy transfer (LET) than protons. This means they deposit more energy in a smaller area, potentially leading to better tumor cell kill rates, especially for radioresistant tumors.

The primary mechanism by which particle therapy works is by ionizing the cells in the targeted area, leading to DNA damage in the tumor cells. The charged particles interact with the atoms in the tissue, causing a disruption in the molecular structure, which leads to cell death. The characteristics of the particles (such as their mass, charge, and energy) influence how they interact with the tissue:
- Protons are lighter and have lower LET compared to helium ions but benefit from the Bragg peak, providing a highly localized dose distribution in the tumor. This makes proton therapy ideal for treating tumors located near critical structures.
- Helium ions (He-4): Helium ions are heavier and carry more charge, meaning they have a higher LET, leading to greater cell-killing power. They are particularly effective for tumors that are resistant to conventional photon therapy, as the increased LET enhances the ability to break through dense, resistant tumor cell walls. They also have a similar Bragg peak effect, which ensures precise targeting of the tumor with minimal damage to surrounding healthy tissue.

Particularly, proton therapy is the most advanced form of radiotherapy and offers new opportunities for improving cancer care and health. In recent years, the number of proton therapy centres has increased considerably in several countries around the world. Currently, there are 120 proton therapy centres globally and new centres are expected to be built in the coming years.

The main benefit of the use of protons instead of X-rays, gamma rays or electrons is the physical properties of the proton beam itself. During proton therapy, interacting protons activate atoms in the tissue and produce positron-emitting isotopes in a variety of reactions with varying energy and probability thresholds. On the other hand, protons penetrate the tissue to a limited depth and deposit most of their energy at a specified area the end of the beam, named Bragg Peak. This is the key benefit of proton therapy, as it makes it possible to target the tumor cells more precisely and reduce irradiation in healthy tissue. For this reason, proton therapy is applied as an alternative to gamma radiotherapy in situations where the tumor to be treated is close to organs at risk sensitive to gamma radiation (brain, heart, etc.).

However, due to the steep dose gradient at the distal edge of the Bragg peak, uncertainties in determination of this range can have a profound impact on the applied dose distribution.

Moreover, although proton therapy is a special form of radiotherapy that uses protons allowing for a very precise deposition of a highly concentrated dose in the tumor, this fact can become disadvantageous if, due to poor positioning of the patient or displacements of internal organs, the proton beam is not adequately directed to the tumor mass with high precision.

Consequently, the verification of the proton therapy dose administered to the patient immediately after each session would be beneficial for the patient, the procedure security, and the clinical goal.

However, there is an unmet medical need of finding reliable techniques aimed at measuring and/or verifying the radiation dose received by a patient after proton therapy treatment. It is essential to verify the proton dose received by each patient after or at least before the next session, as it will be beneficial for the patient, the procedure security and the clinical goal.

The present invention is focused on solving this technical problem and an innovative approach aimed at measuring and/or verifying the radiation dose received by a patient after proton therapy treatment is herein provided.

### DESCRIPTION OF THE INVENTION

### Brief description of the invention

The present invention refers to a method and/or to a system configured to measure and/or verify the radiation dose received by a patient after particle therapy treatment.

Particularly, the present invention provides a computer-implemented method or system for accurately measuring and/or verifying the radiation dose received by a patient following particle therapy treatment. The method utilizes PET imaging of ion-irradiated tissues, such as those treated with protons or helium ions (He-4), to generate a dose map. The invention leverages naturally occurring isotopes in the tissue as sources of F-18 production to ensure accurate and reliable dose estimation.

In one embodiment, the invention involves processing PET images of tissues irradiated by protons or helium ions, with a focus on the interval of F-18 activity dominance (preferably between 60- and 150-minutes post-radiation). The method then generates and outputs a map of the total radiation dose absorbed by the tissue.

The invention is characterized by the fact that the target isotopes for F-18 production are naturally occurring isotopes found in the tissue, such as oxygen-18 (O-18 of 0.2% abundance in natural oxigen) or nitrogen-14 (N-14), which eliminate the need for artificially enriched materials and provide a more accurate and accessible method for dose verification.

Although, such as it is explained above, particle therapy has different advantages, the correct administration of this therapy is of vital importance.

Particularly, the verification of the dose of protons received by the patient after, or at least before the next session, is beneficial for the patient since it improves safety of the procedure and the clinical objective.

During the proton therapy process, the interacting protons activate atoms in the tissue and produce positron-emitting isotopes in a variety of reactions with different energy and probability thresholds (cross-section).

In a particular embodiment, the inventors of the present invention propose that the verification of the dose administered to the patient can be carried out by Positron Emission Tomography (PET), since the presence of these positron-emitting isotopes can be measured by PET. PET detects gamma rays produced when positrons emitted by an isotope interact with electrons in the tissue.

Particularly, the inventors of the present invention have studied the use of the ¹⁸O(p,n)¹⁸F reaction with a half-life of the fluorine-18 (¹⁸F) product of 109.7 min, thus allowing a PET scan 60-150 min after the end of therapy on a nearby PET scanner. The challenge faced was the low abundance of oxygen-18 (¹⁸O) and the low activity produced in patients.

The inventors of the present invention propose the use of F-18 as source of positrons with the intention of designing a reliable strategy to measure and/or verify the radiation dose received by a patient after proton therapy treatment. F-18 is a fluorine isotope which is an important source of positrons. It has a mass of 18.0009380(6) u and its half-life is 109.771(20) minutes, allowing PET 60-150 min after the end of therapy in a nearby high-precision PET scanner. In this regard, it is also interesting that, when F-18 is produced forms the [¹⁸ F]F- ion that remains in the tissue water, not as a gas, so the detection of F-18 is easier.

The concentration of F-18 reproduces the dose profile and, moreover, F-18 dominates approximately 60 minutes after treatment. Although F-18 reproduces the most suitable dose profile, a low amount of F-18 is produced under proton therapy. Consequently, in order to balance the very few F-18 nuclei produced, the inventors of the present invention propose its use for instance through the development of a PET equipment based on bismuth germanate (BGO) with high performance to detect low activities as it does not contain Lu-176 radioactive natural isotope of lutetium present in LSO and LYSO crystals used in PET detectors.

Experimental data was acquired by the inventors of the present invention with a dedicated PET system (DeepBreast) which confirms the feasibility of detecting concentrations of 10 Bq/mL ¹⁸F-FDG (fluorodeoxyglucose).

So, as a proof-of-concept, the present invention could be involved in the clinical practice following a procedure comprising for instance the following steps:
1) Proton therapy treatment planning with a dose map plan for a particular tumor expressed in Gy (J/kg),
2) Irradiating a patient in a proton therapy center according to the above treatment plan in a foreseen session,
3) Obtaining a PET image by using F-18 as source of positrons, optionally combined with computed tomography (CT) or magnetic resonance imaging (MRI). A PET scan measures important body functions, such as metabolism. CT imaging uses special x-ray equipment, and in some cases a contrast material, to produce multiple images of the inside of the body. Combined PET/CT scanners perform almost all PET scans today.
4) Determination of the dose map in Gy in the patient according to the recalculated image.

Nowadays, there is no method of *in vivo* patient dose verification for particle therapy. The advantages it brings with respect to the current state of the art is the *in vivo* verification of the dose received by the patient. *In vivo* verification can provide information on the dose received by organs at risk, especially if they are moved by respiration, cardiac functions or simply by patient movement.

Finally, it is important to consider that F-18 is produced departing from O-18 or N-14. For instance, through a nuclear reaction, protons convert O-18 into F-18, that emits positrons used as a marker in PET studies. In fact, it is precisely the low availability of O-18 in tissues (0.205%) which leads to a low amount of F-18 under proton therapy. On the other hand, F-18 is produced via a nuclear reaction in which helium ions (He-4) interact with N-14.

Of note, in the context of the present invention, there is no need to administer external target isotope for F-18 production (for example N-14 or O-18) into the irradiated tissue. So, the tissue is not modified or disturbed with added amounts of N-14 or O-18. Conversely, in the context of the present invention, the F-18 is produced departing only from N-14 or O-18 already present in the tissue, thus giving a real indication of the proton therapy treatment dose received by the patient. If external N-14 or O-18 were injected with the intention of increasing the signal, this signal would be "artificially" enhanced in those tissues where the N-14 or O-18 is administered, increasing the noise for the dose map reconstruction, and therefore decreasing the signal-to-noise ratio (SNR). Moreover, the injection of external N-14 or O-18 is difficult to control in terms of concentration, therefore the information of dose is disrupted.

The invention provides a computer-implemented method and a system for measuring and verifying the radiation dose received by a patient after particle therapy treatment, utilizing PET images and the natural production of F-18 from isotopes present in the tissue.

Consequently, the first embodiment of the present invention refers to a method for measuring radiation dose (general).

Particularly, the invention provides a method for measuring and/or verifying the radiation dose received by a patient after particle therapy treatment. The method comprises the following steps:
a) Receiving PET images of an ion-irradiated tissue, where the PET images are obtained during the time interval when F-18 activity is dominant in the irradiated tissue, typically between 60 and 150 minutes after radiation.
b) Processing the received PET images to analyze the F-18 distribution.
c) Providing and outputting a total dose map based on the analysis of the PET images.

The method is characterized in that the source of F-18 is a naturally occurring target isotope (e.g., oxygen-18 or nitrogen-14) within the tissue, rather than an externally administered isotope.

The second embodiment of the present invention refers to a method particularly focused on proton therapy.

Particularly, the invention provides a method specifically for verifying the radiation dose received by a patient after proton therapy treatment. This method comprises:
a) Receiving PET images of proton-irradiated tissue, obtained during the time of F-18 activity dominance (between 60 and 150 minutes after radiation).
b) Processing the images to determine the F-18 distribution.
c) Providing and outputting a map of the total dose received by the tissue.

The method is characterized in that the source of F-18 is naturally occurring oxygen-18 (O-18) within the irradiated tissue.

The third embodiment of the present invention refers to a method particularly focused on helium ion therapy.

So, in yet another embodiment, the invention provides a method for measuring the radiation dose received by a patient after therapy with helium ions (He-4). The method comprises:
a) Receiving PET images of helium ion-irradiated tissue, obtained during the interval of F-18 activity dominance.
b) Processing the received images to determine the F-18 distribution.
c) Providing and outputting a total dose map based on the F-18 distribution.

The method is characterized in that the source of F-18 is naturally occurring nitrogen-14 (N-14) within the irradiated tissue.

In all embodiments, the method is characterized by the fact that the source of F-18 (for instance N-14 or O-18) is not externally or artificially administered to the irradiated tissue. This reduces the complexity of the procedure and ensures that the F-18 production comes solely from naturally occurring isotopes within the patient's body.

The method can be particularly useful for patients suffering from cancer, such as brain tumors. The system allows for precise monitoring of the radiation dose delivered to the tumor tissue, ensuring that the correct amount of radiation is applied.

The fourth embodiment of the present invention refers to a system for measuring and/or verifying the radiation dose received by a patient after particle therapy treatment. The system comprises:
a) A processing unit configured to receive and process PET images of ion-irradiated tissue, analyze F-18 activity, and output a total dose map.
b) The system is characterized in that it utilizes naturally occurring isotopes (for instance O-18 or N-14) as the source of F-18 production.

The fifth embodiment of the present invention refers to a system for measuring and/or verifying the radiation dose received by a patient after proton therapy treatment. The system is configured specifically for proton therapy. It receives PET images of proton-irradiated tissue, processes them to analyze F-18 activity, and outputs a dose map. The system uses naturally occurring O-18 within the tissue to produce F-18.

The sixth embodiment of the present invention refers to a system for measuring and/or verifying the radiation dose received by a patient after helium ion treatment. The system is configured for helium ion therapy. It operates in a similar manner, using N-14 as the naturally occurring isotope to produce F-18 from helium ion irradiation.

The present invention also comprises additional embodiments:

### • PET Scanner Integration:

The system may further comprise a PET scanner with a planar geometry defined by parallel detector planes or composed of BGO scintillators. This allows for detailed imaging of the irradiated tissue and accurate dose mapping.

### • Computer Program and Storage Medium

A computer program containing instructions to execute the above methods is also provided. This program can be stored on a computer-readable medium, allowing the method to be executed on a system for radiation dose verification.

The present invention is associated with several advantages, such as:
- Real-time radiation dose verification: The use of PET imaging to verify the radiation dose during therapy allows for real-time monitoring.
- Natural isotope use: By using naturally occurring isotopes (O-18, N-14) as sources of F-18, the method eliminates the need for externally administered isotopes, simplifying the process.
- Versatility: The method and system are adaptable to different types of particle therapy, including proton and helium ion treatments.
- Cancer treatment application: Particularly beneficial for patients undergoing cancer treatment, such as those with brain tumors, ensuring accurate dose delivery to the target tissue.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of". Thus, the phrase "consisting of" indicates that the listed elements are required or mandatory, and that no other elements may be present.
- A "target isotope for F-18 production" refers to a naturally occurring or stable isotope present in a tissue or material that, when exposed to radiation (such as ion beams or particle therapy), undergoes a nuclear reaction resulting in the production of fluorine-18 (F-18). The target isotope serves as the precursor or source material from which F-18 is generated. In this context, the term specifically includes isotopes that are part of the natural composition of biological tissues, such as oxygen-18 (O-18) or nitrogen-14 (N-14), which, when irradiated, can produce F-18 through nuclear processes (such as alpha particle bombardment or neutron capture). These target isotopes are not externally administered or artificially introduced into the tissue, but rather occur naturally within the patient's body, allowing for non-invasive monitoring and verification of radiation dose delivery through the production of F-18.

### Brief description of the figures

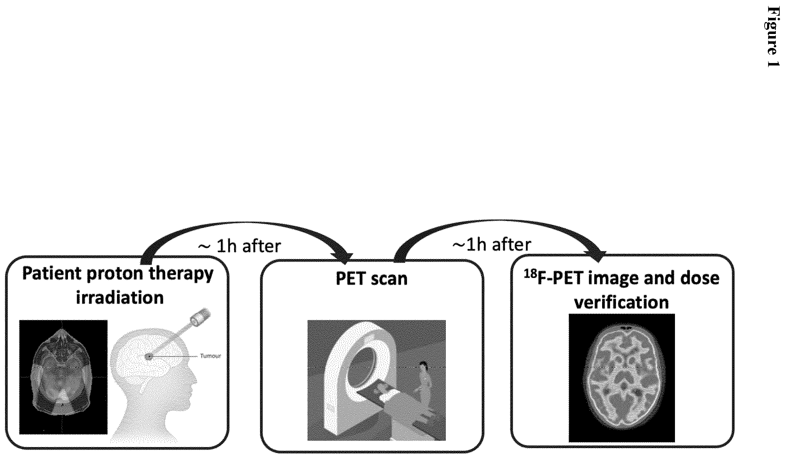

**Figure 1****.** Block diagram of the dose verification strategy proposed in the present invention.
**Figure 2****.** Top: dose (Gy) in 3×3×3 cm³ cube with 5×10⁹ protons reaching 0.74 Gy. Middle ¹⁸F initial activity map with 100 Gy dose reaching 2.2 Bq/ml. Bottom: ¹¹C initial activity map with 100 Gy dose reaching 9.6 kBq/ml. Note reproduction of dose with ¹⁸F and range plus dose error with ¹¹C.
**Figure 3****.** Reconstructed images of the vial at different concentration values and reconstructed image of the background.
**Figure 4****.** Activity concentration measured in BGO-based clinical scanner of a syringe of 30 mL of F 18]fluorodeoxyglucose. Left: activity concentration of 1 kBq/mL, center 1 Bq/mL (corresponding to 10 Gy after 210 min from treatment in brain tissue), right 0.3 Bq/mL (3 Gy after 210 min).

### Detailed description of the invention

The following Examples are provided with the objective of illustrating the invention and without the intention of limiting its scope of protection.

### Example 1. Material and methods

### Example 1.1. Simulations

Simulations of the proton dose received in a 3×3×3 cm³ (27 mL) tumor phantom in brain tissue have been developed using Gate (version 9.3). First, 5×10⁹ protons were simulated, resulting in a maximum dose of 0.74 Gy. This simulation has already produced 55516 ¹⁸F isotope atoms per mL assuming 50% abundance of ¹⁸O and ¹⁶O. Then, a recalculation was performed for a dose of 100 Gy, typically used in proton FLASH and the natural ¹⁸O abundance of 0.205%. In this case, the activity maps of ¹¹C and ¹⁸F were determined. Note, that these maps of activities are generated, and they are not a simulated PET image.

### Example 1.2. Preliminary experimental acquisitions

Experimental data was acquired using the dedicated *DeepBreast* PET system. The scanner has an inner diameter of 200 mm, and an outer diameter of 225 mm, with an axial length of 50 mm. The system consists of 14 curved LYSO crystals coupled to custom flexible PCBs with SiPM arrays of 12×12 elements (3×3 mm² active area). Each PCB contains a row and column reduction readout electronic. The data acquisition system consists of 14 analog to digital converter boards (ADCs), a trigger unit (coincidence window of 3 ns) and the acquisition computer. The x- and *y*- annihilation photon impact coordinates were estimated using a Neural Network (NN) technique. The energy and Depth of Interaction (DOI) were calibrated using Voronoi diagrams. The continuous DOI information was included to correct for the parallax error. The images were reconstructed using MLEM method with an isotropic voxel size of 0.5 mm³ and no corrections were applied to the images.

Preliminary experimental data was acquired by using a bottle filled with 20.25 mL solution of ¹⁸F-FDG with an initial activity concentration of 0.199 MBq/mL until a final concentration of 0.73 Bq/mL. Moreover, a background measurement was acquired. The data was reconstructed in 1-hour windows with 10 iterations. Another set of measurements was done on commercial Albira two ring microPET/CT system with LYSO crystals.

In another preliminary experiment we have checked if a clinical scanner with BGO crystal detectors, which does not contain radioactive Lu-176 is capable of detecting even lower activity concentrations without noise related to this detector radioactivity. The experiment was done using 30 mL solution of ¹⁸F-FDG with an initial activity concentration of 1 kBq/mL until a final concentration of 0.3 Bq/mL.

### Example 2. Results

### Example 2.1. Simulations

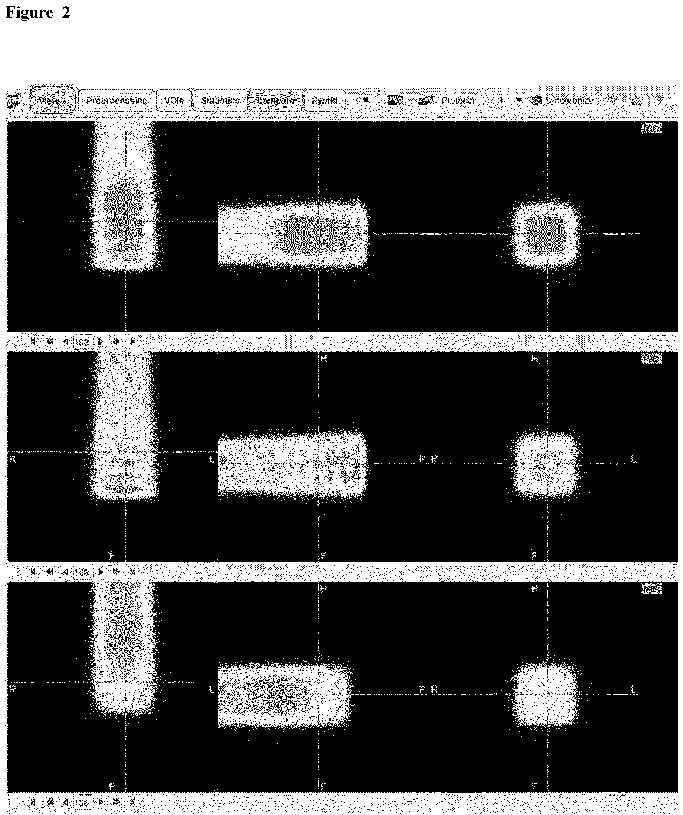

**Figure 2** (top) shows the simulated dose in 3×3×3 cm³ cube with 5×10⁹ protons reaching 0.74 Gy. **Figure 2****,** middle and bottom, show the maps of activities for ¹⁸F and ¹¹C, respectively, for a dose of 100 Gy in the simulated tumor volume. The equivalent ¹⁸F concentration is 8.6 Bq/mL, very close to the detection limit of PET.

### Example 2.2. Preliminary experimental acquisitions

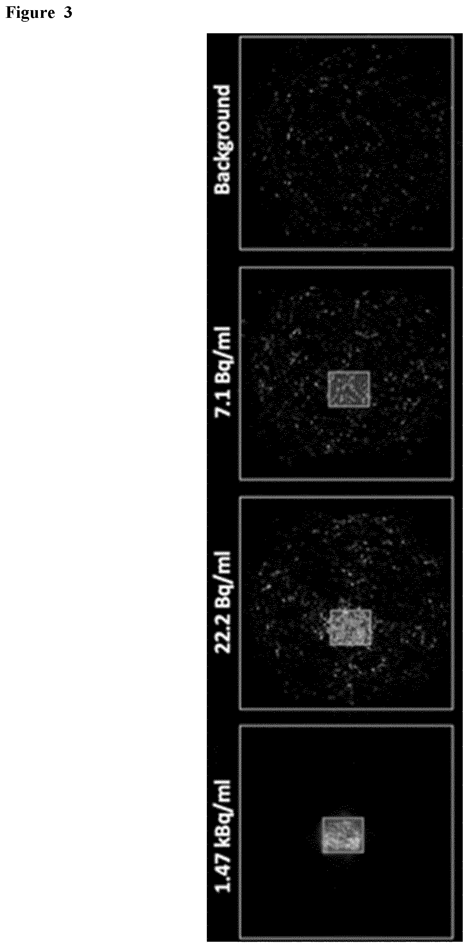

**Figure 3** shows the reconstructed images of the vial at concentration values of 1.47 kBq/ml, 22.2 Bq/ml and 7.1 Bq/ml and also the reconstructed image for the background measurement. The image quality degrades considerably for concentration values less than ~7 Bq/ml. This could be due to the noise related to intrinsic radioactive activity of the ¹⁷⁶Lu from the LYSO crystals that make up the *DeepBreast* system (see **Figure 2****).** Preliminary assessment done with background in the Albira system indicates that within the energy window of the PET system there is about 86 counts per second per mL of LYSO crystal, which is consistent with 456 decays per second of ¹⁷⁶Lu in LSO crystal.

We compared the signal to noise ratio of the images dividing the volume with activity concentration outside of the bottle with activity inside the bottle. Even for lowest activity of 0.6 Bq/mL the S/N was about 1.5.

In conclusion, the present invention proposes a method and/or system for dose verification in proton therapy by acquiring a PET scan (measuring the gamma rays resulting from the decay of the generated ¹⁸F) 1 hour after the end of therapy.

Simulations have been performed and demonstrate that the ¹⁸F reproduces the most suitable protons dose profile.

Moreover, preliminary experimental results with the *DeepBreast* system show the feasibility of detecting 10 Bq/mL ¹⁸F-FDG solution. This value could be improved using a shorter coincidence window (1 ns instead of 3 ns) that would reduce random signals due to ¹⁷⁶Lu or using a PET scanner based on BGO. These two options are being taken into consideration to improve results.

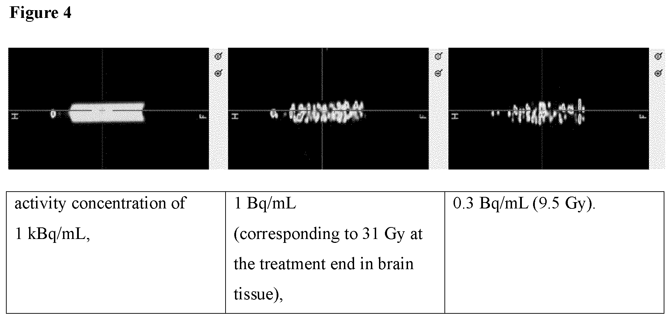

Finally, the activity concentration measured in a BGO-based clinical scanner for a syringe containing 30 mL of F-18 fluorodeoxyglucose (FDG) is presented in **Figure 4****.** The figure shows three different activity concentrations:
- On the left, the activity concentration is 1 kBq/mL.
- In the center, the activity concentration is 1 Bq/mL, which corresponds to a radiation dose of 10 Gy after 210 minutes in brain tissue.
- On the right, the activity concentration is 0.3 Bq/mL, resulting in a radiation dose of 3 Gy after 210 minutes.

These results demonstrate the relationship between activity concentration and radiation dose over time in brain tissue following FDG administration.

## Claims

1. A computer-implemented method for measuring and/or verifying the radiation dose received by a patient after particle therapy treatment, comprising the steps:
a) Receiving one or more PET images of an ion-irradiated tissue, wherein the PET images have been obtained in the interval of F-18 activity dominance in said tissue after said radiation, preferably between 60 and 150 minutes after said radiation;
b) Processing the images received according to the step a);
c) Providing and output with map of total dose;
**characterized in that** the target isotope for F-18 production which is present in the tissue as a source of F-18 is only a naturally occurring target isotope for F-18 production.

2. A computer-implemented method, according to claim 1, for measuring and/or verifying the radiation dose received by a patient after proton therapy treatment, comprising the steps:
a) Receiving one or more PET images of a proton-irradiated tissue, wherein the PET images have been obtained in the interval of F-18 activity dominance in said tissue after said radiation, preferably between 60 and 150 minutes after said radiation;
b) Processing the images received according to the step a);
c) Providing and output with map of total dose.
**characterized in that** the O-18 which is present in the tissue as a source of F-18 is only naturally occurring O-18.

3. A computer-implemented method, according to claim 1, for measuring and/or verifying the radiation dose received by a patient after therapy treatment with helium ions (He-4), comprising the steps:
a) Receiving one or more PET images of a helium ions (He-4)-irradiated tissue, wherein the PET images have been obtained in the interval of F-18 activity dominance in said tissue after said radiation, preferably between 60 and 150 minutes after said radiation;
b) Processing the images received according to the step a);
c) Providing and output with map of total dose;
**characterized in that** the N-14 which is present in the tissue as a source of F-18 is only naturally occurring O-18.

4. A computer-implemented method, according to any of the previous claims, **characterized in that** N-14 or O-18 is not externally or artificially administered to the irradiated tissue.

5. A computer-implemented method, according to any of the previous claims, for measuring and/or verifying the radiation dose received by a patient suffering from cancer, preferably suffering from a brain tumour.

6. System configured to measure and/or verify the radiation dose received by a patient after particle therapy treatment which comprises a processing unit configured to:
a) Receive PET images of an ion-irradiated tissue, wherein the PET images have been obtained in the interval of F-18 activity dominance in said tissue after said radiation, preferably between 60 and 150 minutes after said radiation;
b) Process the images received according to the step a);
c) Provide and output with map of total dose;
**characterized in that** the target isotope for F-18 production which is present in the tissue as a source of F-18 is only a naturally occurring target isotope for F-18 production.

7. System, according to claim 6, configured to measure and/or verify the radiation dose received by a patient after proton therapy treatment which comprises a processing unit configured to:
a. Receive PET images of a proton-irradiated tissue, wherein the PET images have been obtained in the interval of F-18 activity dominance in said tissue after said radiation, preferably between 60 and 150 minutes after said radiation;
b. Process the images received according to the step a);
c. Provide and output with map of total dose;
**characterized in that** the O-18 which is present in the tissue as a source of F-18 is only a naturally occurring O-18.

8. System, according to claim 6, configured to measure and/or verify the radiation dose received by a patient after therapy treatment with helium ions (He-4) which comprises a processing unit configured to:
a. Receive PET images of a helium ions (He-4)-irradiated tissue, wherein the PET images have been obtained in the interval of F-18 activity dominance in said tissue after said radiation, preferably between 60 and 150 minutes after said radiation;
b. Process the images received according to the step a);
c. Provide and output with map of total dose;
**characterized in that** the N-14 which is present in the tissue as a source of F-18 is only a naturally occurring N-14.

9. System, according to any of the claims 6 to 8, **characterized in that** N-14 or O-18 is not externally or artificially administered to the irradiated tissue.

10. System, according to any of the claims claim 6 to 9, configured to measure and/or verify the radiation dose received by a patient suffering from cancer, preferably suffering from a brain tumour.

11. System, according to any of the claims 6 to 10, further comprising a PET scanner which has a planar geometry defined by parallel detector planes or composed of BGO scintillators.

12. A computer program comprising instructions to cause the system of claims 6 to 11 execute the steps of the method of claims 1 to 5.

13. A computer-readable medium having stored thereon the computer program of claim 12.
